# EUROPEAN PATENT SPECIFICATION

(11) **EP 0 589 437 B1**
(45) Date of publication and mention of the grant of the patent: **07.04.1999**
(21) Application number: 93115290.4
(22) Date of filing: 22.09.1993
(51) Int. Cl.: B32B 27/00, A61F 13/15, B32B 27/32, B32B 27/36, B32B 27/40

(54) **Compostable disposable personal care articles**
Wegwerfbare kompostierbare Hygieneartikel
Articles d'hygiène compostables à jeter

(30) Priority: 22.09.1992 US 949279
(43) Date of publication of application: 30.03.1994
(73) Proprietor: KIMBERLY-CLARK WORLDWIDE, INC., Neenah, Wisconsin 54956 (US)
(72) Inventor: Haffner, William Bela, Kennesaw, Georgia 30144 (US); Pazdernik, Patrick Alan, Neenah, Wisconsin 54956 (US); Cappel, Jennifer Brita, Fond du Lac, Wisconsin 54935 (US); Garrett, Jr., Lance James, Marietta, Georgia 30062 (US); Hetzler, Connie Lynn, Neenah, Wisconsin 54956 (US)
(74) Representative: DIEHL GLAESER HILTL & PARTNER

(56) References cited:
- EP-A- 0 254 468
- GB-A- 2 193 925
- US-A- 4 894 291
- DATABASE WPIL, no. 88-054 273 DERWENT PUBULICATIONS LTD., London, GB

## Description

The present invention relates to disposable personal care articles, comprising compostable films.

Disposable personal care articles, such as disposable diapers, utilize a variety of materials, including an absorbent core material for absorbing and retaining targeted body fluids, such as urine, a liner material which contacts the body of the user and permits transfer to the absorbent core material of the body fluids to be absorbed, and a backsheet which prevents leakage and confines the absorbed body fluids within the article. The function of the backsheet requires materials which can withstand fluid insults during normal use of the articles. A typical diaper backsheet material is polyethylene, which is an economical waterproof material which functions very well for its intended purpose.

However, increasing emphasis today is being placed on making products which are more environmentally friendly. Biodegradable, flushable, compostable, or otherwise recoverable materials are being sought as replacements for conventional materials. UK Patent GB2193925 relates to ostomy bags and incontinence bags which are made of a WC -disposable sheet material. This sheet material comprises a water soluble polvinyl-alcohol film which is laminated to a water insoluble PE or PVC film. Japanese Patent JP-63-11338 discloses a high gas-barrier transparent laminated plastic film comprising a polyvinylalcohol series resin film containing 50 mole % or more of polyvinyl alcohol and laminated on at least one face thereof, a transparent plastic film having an excellent steam barrier property, wherein the laminated faces of the two films are adhered with an adhesive which is a binary system urethanic adhesive and in which a ratio of a hardener component to an adhesive component is 30 to 70 % by weight. EP-A-254-468 discloses a gas barrier structure comprising a base synthetic thermoplastic polymeric layer, e.g. nylon or polyethylene, and having two coatings on one side of the base layer. The first coating is a solvent-based urethane primer and the second coating comprises a polyvinyl alcohol gas barrier material. The primer is present in the range of 0.3 to 3.0 g/m² and the polyvinyl alcohol is present in an amount of up to 2.0 g/m². The polyvinyl alcohol is deposited from solution or dispersion.

In the case of products such as disposable diapers, finding a backsheet material to replace polyethylene could be environmentally advantageous if such a material would render the diaper compostable without sacrificing the agueous barrier properties required of the material in use.

It is therefore the object of the present invention to provide care articles without the above mentioned drawbacks. This object is solved by the care articles according to independent claim 1. Further advantageous features, aspects and details of the invention are evident from the dependent claims, the description and the examples.

According to the invention, there are provided compostable personal care articles having a functionally waterproof backsheet without significant, if any, loss in performance which can be made with a backsheet comprising a layered film material which is waterproof for a time sufficiently long to perform its intended purpose, but which disassociates and degrades when subjected to composting conditions.

Accordingly, the invention resides in a disposable personal care article suitable for composting, comprising a body-side liner, a backsheet, and an absorbent core sandwiched in between the liner and the backsheet, wherein the backsheet comprises a compostable layered film having a base layer of polyvinyl alcohol, said base layer coated by a pinhole-free, crack- and abrasion-resistant layer of a water-resistant material disposed on the surface of the backsheet facing or in contact with the absorbent core and an intermediate layer of a primer material, whereby said primer material is dried and cured after applying said primer material on said base layer or said layer of water-resistant material and before sandwiching said primer material between said base layer and said layer of water-resistant material, said layer of water-resistant material being adhered to the polyvinyl alcohol layer sufficiently to pass the Adhesion Test performed in accordance with a modified ASTM D3330-90 standard test using Method A and selected from the group consisting of polyvinylidene chloride, polyvinyl chloride, polyvinyl dichloride, cellulose nitrate, polyethylene, ethylene acrylic acid, ethylene vinyl alcohol, and combinations and copolymers thereof.

A compostable layered film comprising a base layer of polyvinyl alcohol and an outer layer of a water resistant material, such as hereinafter described, is sufficiently adhered to the polyvinyl alcohol layer to provide adequate integrity for its intended purpose. A coating or layer of a primer material on the surface of the polyvinyl alcohol base layer is necessary to provide sufficient adhesion of the water resistant materials to the polyvinyl alcohol base layer. Preferred are embodiments where the water resistant layer is polyvinylidene chloride.

A disposable personal care article suitable for composting comprises a body-side liner, a layered backsheet, and an absorbent core sandwiched between the liner and the backsheet, wherein the layered backsheet comprises a base layer or film of polyvinyl alcohol having a water-resistant layer disposed on the surface of the backsheet facing or in contact with the absorbent core and adhered to the polyvinyl alcohol film sufficiently by means of a primer material to pass the Adhesion Test (hereinafter defined). Preferably the water-resistant coating or layer is on both sides of the polyvinyl alcohol base layer. A suitable means for providing adequate adhesion is the addition of a layer of a primer material to the polyvinyl alcohol film. A water-resistent layer of polyvinylidene chloride adhered with a primer to at least one side of the polyvinyl alcohol layer is preferred.

In a particular embodiment of the invention the backsheet comprises a three layered film having a base layer of polyvinyl alcohol and two outer layers of maleic anhydride-grafted polyethylene.

Disposable personal care articles within the scope of this invention include e.g. disposable diapers, feminine pads, incontinence garments and training pants. Such articles having a backsheet, an absorbent core and a bodyside liner are common commercial products well known in the art. The specific product constructions do not form a part of this invention other than to the extent that they have the three elements set forth herein.

The polyvinyl alcohol (PVOH) film or base layer useful for purposes herein must have sufficient strength properties to perform the intended function of the layered film of this invention. Suitable characteristics include: a tensile strength of at least about 500 grams per 2.54 cm (about 500 grams per inch) of width in both the cross-machine direction (CD) and the machine direction (MD) orientation, preferably at least about 700 grams, and more preferably from about 700 to about 3000 grams; an elongation-at-break, as measured by ASTM test D 882, of at least about 25 percent, preferably from about 25 to about 200 percent; and a minimum dart impact strength, as measured by ASTM test method D 1709-85, Method A, of about 40 grams or greater, preferably about 60 grams or greater. In general, a preferred polyvinyl alcohol resin for making films for use in accordance with this invention has a relatively high molecular weight in order to obtain better sheet formation. Such resins also preferably contain a plasticizer, such as glycerin, to provide a more flexible, less brittle film. The thickness of the PVOH film can be from about 12.7 to about 76.2 µm (about 0.5 to about 3 mils), preferably the thickness is about 25.4 µm (about 1 mil (0.001 inch)). Suitable commercially available PVOH film materials include solution cast PVOH film made by Mono-Sol Inc., Gary, Indiana and film from melt-processable extruded PVOH resin made by Air Products, Inc., Allentown, Pennsylvania, such as Vinex 2034™ resin or modifications thereof.

The water-resistant coating or layer must be pin hole-free, crack and abrasion resistant, and able to form a moisture barrier on the PVOH film or layer. It can be applied to the PVOH film from solutions or dispersions (suspensions), such as by printing. The thickness of the water-resistant coating can be from about 1.27 to about 25.4 µm (about 0.05 mil to about 1 mil), preferably from about 2.54 to about 12.7 µm (about 0.1 to about 0.5 mil). Any liquid and moisture barrier coating for film application with acceptable performance properties can be used, including polyvinylidene chloride, polyvinyl chloride, polyvinyl dichloride, polyethylene, cellulose nitrate, ethylene acrylic acid, and ethylene vinyl alcohol, including combinations and copolymers thereof. Copolymers may possess less crystallinity and thus be less stiff or more flexible and easy to dissolve, which can enhance their processability. Preferred water-resistant coatings are the saran-type polyvinylidene chlorides such as F-239, F-278 and F-310, available from Dow Chemical Company, Midland, Michigan.

The primer film or coating must have suitable adhesion to the PVOH film or base layer and to the water-resistant layer as measured by the Adhesion Test (described in the hereafter). The primer film should also be crack resistant and preferably be biodegradable. Preferable materials to be used to make the primer film or coating include crosslinkable polyurethanes or crosslinkable polyesters, including aliphatic polyesters such as polycaprolactone, which have the appropriate surface energy characteristics to adhere to the polyvinyl alcohol substrate when formed into a coating or layer. Commercially available primer materials include Adcote 710™ polyester from Morton International, Chicago, Illinois, or Bostik 7650™ polyurethane, a crosslinkable polyester which can be crosslinked using an isocyanate crosslinking agent, from Bertek, Inc., St. Albans, Vermont. The primer material can be applied by coating from solution with subsequent drying and curing steps to yield as close to a monomolecular layer as possible. Suitable means include gravure, flexographic, lithographic, spray, or extrusion coating methods. The thickness of the primer coating can be minimal, provided it is continuous, preferably from about 1.27 to about 2.54 µm (about 0.05 to about 0.1 mil).

The Adhesion Test is a measure of the degree of adhesion between the polyvinyl alcohol film and the water-resistant layer. In order to have suitable adhesion to pass the Adhesion Test, there must be sufficient adhesion to retain integrity during use. In particular, the Adhesion Test is determined by applying standard Scotch Brand #610 tape to the water repellant coating and peeling the tape off rapidly to determine if any separation of the coating has taken place. The Adhesion Test method used to determine if the coating/primer has the necessary bond strength to the base PVOH film is a modified ASTM D3330-90 standard test using Method A. Samples are prepared according to the procedure, using the following modifications: a Scotch Brand #610 test tape available from 3M Company, St. Paul, Minnesota, a test specimen of coated film 10.16 cm by 15.24 cm (four inches by six inches) where the longer dimension is the machine direction of the film, and no test panel. The test procedure is modified to have a piece of test tape cut 75 mm long and 19 mm wide with a kraft paper leader strip attached to one end of the test tape with about 25 mm of the kraft paper in contact with the test tape. The exposed 50 mm of test tape is placed on the coated surface of the test sample, the test tape being centered on the sample with the longer edge of the test tape parallel to the machine direction (longer dimension) of the sample. The test tape is rolled down according to ASTM D3330-90. A high-speed peel separation rate of 6.1 m/min (240 in./min.) is used to determine if any delamination occurs between the coating and the PVOH film. The test method is a pass/fail procedure and does not measure a peel force. Tests should be performed to determine adequate bonding in both in the machine and cross machine directions. If there is any separation, the coating fails the test. If there is no separation, the coating passes the test.

### Examples

### Comparative Example: (Polyvinylidene Chloride/PVOH)

A composite film of polyvinylidene chloride and PVOH was prepared by using a 25.4 µm (one mil) solution cast PVOH base film from Specialty Products (McAdoo, Pennsylvania), and a Dow F-310 saran by coating through an MEK (methylethyl ketone) solution using a gravure cylinder on a commercial coating line to give an approx. 2.54 µm (approximate 1 mil) coating. The coating was dried in an in-line hot air oven at 37.8 to 48.9 °C (100-120°F) for about twenty seconds. The composite film was allowed to cool to ambient and samples were then taken for coating adhesion testing. The composite film was subjected to the Adhesion Test as described above and failed.

### Example 1: (Polyvinylidene Chloride/Primer/PVOH)

A composite film consisting of a PVOH base, a tie coating of a cross-linked polyurethane primer, and a top coat of polyvinylidene chloride was prepared by using a 25.4 µm (1 mil) solution cast PVOH base film from Specialty Products and first coating it with a primer consisting of a Bostik 7650™ crosslinked polyurethane made by mixing Bostik 7650™ and 1 percent Boscodure 22™ isocyanate, with an MEK/toluene mix as a solvent, and applying it to the base PVOH film by a gravure cylinder and then drying it in an in-line, hot air oven at 37.8 to 48.9°C (100-120°F) for about twenty seconds. Film material was then wound up on a roll. Subsequently, the saran (polyvinylidene chloride) coating was applied to the primer-coated film in the same manner described above yielding a polyvinylidene chloride/primer/PVOH composite. The composite film was subjected to the Adhesion Test as described above and passed.

### Example 2: (Composting)

The foregoing sample films were subjected to composting conditions to determine their compostability. More specifically, the composite film produced in Example 2 was subjected to a disposable diaper manufacturing process and as a result several products were produced. Representative products were then placed into a commercial in-vessel composting process in St. Cloud, Minnesota at Recomp, Inc. for composting. The samples were put inside a heavy gauge mesh polyester bag of about 1.3 cm (about one-half inch) mesh which permits moisture and compost to mix with the sample and allow for easy identification. Product samples were recovered following three days of in-vessel composting and analyzed for degradation. Results showed approximately 80 percent total degradation of the composite film after this relatively short duration. Composting conditions were typical for mixed municipal solid waste commercial composting operations.

It will be appreciated that the foregoing examples, given for purposes of illustration, are not to be construed as limiting the scope of this invention, which is defined by the following claims.

## Claims

1. A disposable personal care article suitable for composting, comprising a body-side liner, a backsheet, and an absorbent core sandwiched in between the liner and the backsheet, wherein the backsheet comprises a compostable layered film having a base layer of polyvinyl alcohol, said base layer coated by a pinhole-free, crack- and abrasion-resistant layer of a water-resistant material disposed on the surface of the backsheet facing or in contact with the absorbent core and an intermediate layer of a primer material, whereby said primer material is dried and cured after applying said primer material on said base layer or said layer of water-resistant material and before sandwiching said primer material between said base layer and said layer of water-resistant material, said layer of water-resistant material being adhered to the polyvinyl alcohol layer sufficiently to pass the Adhesion Test performed in accordance with a modified ASTM D3330-90 standard test using Method A and selected from the group consisting of polyvinylidene chloride, polyvinyl chloride, polyvinyl dichloride, cellulose nitrate, polyethylene, ethylene acrylic acid, ethylene vinyl alcohol, and combinations and copolymers thereof.

2. The article of claim 1 wherein the water-resistant layer of the backsheet is polyvinylidene chloride.

3. The article of one of claims 1 or 2 wherein the intermediate layer of a primer material is selected from the group consisting of crosslinked polyesters and crosslinked polyurethanes.

4. The article of one of claims 1 to 3 wherein the thickness of the water resistant layer of the backsheet is from 1.27 to 25.4 µm (0.05 mil to 1 mil), preferably 2.54 to 12.7 µm (0.1 mil to 0.5 mil).

5. The article of one of claims 1 to 4 wherein the thickness of the base layer of the backsheet is from 12.7 to 76.2 µm (0.5 mil to 3 mils) and the intermediate layer of the backsheet is a crosslinked polyurethane primer having a thickness of from 1.27 to 2.54 µm (0.05 mil to 0.1 mil).

6. The article of one of claims 1 to 4 wherein the backsheet comprises a three-layered film having a base layer of polyvinyl alcohol and two outer layers of maleic anhydride-grafted polyethylene.

## Patentansprüche

1. Wegwerfbarer kompostierbarer Hygieneartikel mit einer körperseitigen Ausfütterung, einer Rückschicht und einem saugfähigen Kern, welcher sandwichartig zwischen der Ausfütterung und der Rückschicht angeordnet ist, wobei die Rückschicht eine kompostierbare geschichtete Folie aufweist mit einer Basisschicht aus Polyvinylalkohol, einer auf der Basisschicht aufgebrachten nadelstichfreien, rißbeständigen und abriebfesten Schicht aus wasserbeständigem Material, die auf der dem saugfähigen Kern zugewandten oder diesen berührenden Oberfläche der Rückschicht angeordnet ist, und einer Zwischenschicht aus einem Primermaterial, wobei das Primermaterial nach der Aufbringung des Primermaterials auf der Basisschicht oder der Schicht aus wasserbeständigem Material und vor der sandwichartigen Anordnung des Primermaterials zwischen der Basisschicht und der Schicht aus wasserbeständigem Material getrocknet und ausgehärtet wird, wobei die Schicht aus wasserbeständigem Material an der Polyvinylalkoholschicht derart haftend angebracht ist, daß sie dem Adhäsionstest, welcher gemäß eines modifizierten ASTM-D3330-90-Standardtestverfahrens unter Verwendung der Methode A durchgeführt wird, standhält, und aus der Gruppe bestehend aus Polyvinylidenchlorid, Polyvinylchlorid, Polyvinyldichlorid, Cellulosenitrat, Polyethylen, Ethylenacrylsäure, Ethylenvinylalkohol sowie Kombinationen und Copolymere davon ausgewählt ist.

2. Artikel gemäß Anspruch 1, bei dem die wasserbeständige Schicht der Rückschicht ein Polyvinylidenchlorid ist.

3. Artikel gemäß einem der Ansprüche 1 oder 2, bei dem die Zwischenschicht aus einem Primermaterial ausgewählt ist aus der Gruppe bestehend aus vernetzten Polyestern und vernetzten Polyurethanen.

4. Artikel gemäß einem der Ansprüche 1 bis 3, bei dem die Dicke der wasserbeständigen Schicht der Rückschicht 1,27 bis 25,4 µm (0,05 mil bis 1 mil), vorzugsweise 2,54 bis 12,7 µm (0,1 mil bis 0,5 mil) beträgt.

5. Artikel gemäß einem der Ansprüche 1 bis 4, bei dem die Dicke der Basisschicht der Rückschicht 12,7 bis 76,2 µm (0,5 mil bis 3 mils) und die Zwischenschicht der Rückschicht ein vernetzter Polyurethanprimer mit einer Dicke von 1,27 bis 2,54 µm (0,05 mil bis 0,1 mil) ist.

6. Artikel gemäß einem der Ansprüche 1 bis 4, bei dem die Rückschicht eine dreischichtige Folie mit einer Basisschicht aus Polyvinylalkohol und zwei äußeren Schichten aus mit Maleinsäureanhydrid gepfropftem Polyethylen aufweist.

## Revendications

1. Article d'hygiène intime jetable convenant à la dégradation en compost, comprenant une doublure côté corporel, une feuille support et un noyau absorbant pris en sandwich entre la doublure et la feuille support, dans lequel la feuille support est constituée d'un film stratifié dégradable en compost ayant une couche de base de poly(alcool vinylique), ladite couche de base étant revêtue d'une couche dépourvue de perforation et résistant aux fissures et à l'abrasion, d'un matériau résistant à l'eau disposé sur la surface de la feuille support tournée vers, ou en contact avec, le noyau absorbant et une couche intermédiaire d'un matériau d'apprêt, ledit matériau d'accrochage étant séché et durci après l'application dudit matériau d'accrochage sur ladite couche de base ou ladite couche de matériau résistant à l'eau et avant la prise en sandwich dudit matériau d'accrochage entre ladite couche de base et ladite couche de matériau résistant à l'eau, ladite couche de matériau résistant à l'eau étant collée suffisamment à la couche de poly(alcool vinylique) pour satisfaire au Test d'Adhérence mis en oeuvre selon un test standard ASTM D3330-90 modifié utilisant le Procédé A, et choisi dans le groupe consistant en le poly(chlorure de vinylidène), le poly(chlorure de vinyle), le poly(dichlorure de vinyle), le nitrate de cellulose, le polyéthylène, l'acide éthylène-acrylique, l'alcool éthylène vinylique et des combinaisons et copolymères de ceux-ci.

2. Article selon la revendication 1, dans lequel la couche résistante à l'eau de la feuille support est en poly(chlorure de vinylidène).

3. Article selon l'une des revendications 1 ou 2, dans lequel la couche intermédiaire de matériau d'accrochage est choisie dans le groupe consistant en les polyesters réticulés et les polyuréthanes réticulés.

4. Article selon l'une des revendications 1 à 3, dans lequel l'épaisseur de la couche résistant à l'eau de la feuille support est comprise entre 1,27 et 25,4 µm (entre 0,05 et 1 millième de pouce), de préférence entre 2,54 et 12,7 µm (entre 0,1 et 0,5 millième de pouce).

5. Article selon l'une des revendications 1 à 4, dans lequel l'épaisseur de la couche de base de la feuille support est comprise entre 12,7 et 76,2 µm (entre 0,5 et 3 millièmes de pouce) et la couche intermédiaire de la feuille support est un accrochage formé d'un polyuréthane réticulé ayant une épaisseur comprise entre 1,27 et 2,54 µm (entre 0,05 et 0,1 millième de pouce).

6. Article selon l'une des revendications 1 à 4, dans lequel la feuille support comprend un film à trois couches ayant une couche de base formée de poly(alcool vinylique) et deux couches extérieures de polyéthylène greffées à l'anhydride maléique.
